# EUROPÄISCHE PATENTANMELDUNG

(11) **EP 0 786 520 A1**
(43) Veröffentlichungstag der Anmeldung: **30.07.1997**
(21) Anmeldenummer: 96118979.2
(22) Anmeldetag: 26.11.1992
(51) Int. Cl.: C12N 15/12, C07K 14/48, A61K 38/18, C07K 1/113, C12N 1/21

(54) **Verfahren zur gentechnologischen Herstellung von bioligisch aktivem beta-NGF**

(30) Priorität: 27.11.1991 DE 4139000
(62) Teilanmeldung aus: 92120207.3
(71) Anmelder: BOEHRINGER MANNHEIM GMBH, 68305 Mannheim (DE)
(72) Erfinder: Lang, Kurt, Dr., 82377 Penzberg (DE); bartke, Ilse, Dr., 82347 Bernried (DE); Naujoks, Kurt, Dr., 82377 Penzberg (DE); Rudolph, Rainer, Prof. Dr., 06114 Halle (DE); Stern, Anne, Dr., 82377 Penzberg (DE)

(57) **Zusammenfassung**

Zur gentechnologischen Herstellung von biologisch aktivem β-NGF (Nerve Growth Factor) durch Expression einer entsprechenden DNA-Sequenz in Prokaryonten als Wirtszellen werden die nach Expression dieser DNA-Sequenz in E.coli erhaltenen unlöslichen Aggregate von inaktivem β-NGF durch (1) Solubilisierung, (2) gegebenenfalls Dialyse oder/und Derivatisierung des erhaltenen β-NGF mit der oxidierten Form einer Verbindung, deren reduzierte Form Sulfhydrylgruppen aufweist und deren oxidierte Form ein über eine Disulfidbrücke verbundenes Dimer oder Verbindung ist, zum gemischten Disulfid, und (3) Naturierung mit Hilfe eines Redoxsystems, bestehend aus einer Verbindung in oxidierter oder/und reduzierter Form, deren reduzierte Form Sulfhydrylgruppen aufweist und deren oxidierte Form ein über eine Disulfidbrücke verbundenes Dimer ist, in eine lösliche aktive Form überführt.

## Beschreibung

Die Erfindung betrifft ein Verfahren zur gentechnologischen Herstellung von biologisch aktivem β-NGF durch Expression einer entsprechenden DNA-Sequenz in Prokaryonten als Wirtszellen, rekombinanten modifizierten β-NGF, eine DNA-Sequenz sowie einen Expressionsvektor und die Verwendung des rekombinanten modifizierten β-NGF in einer therapeutischen Zusammensetzung.

Nerve Growth Factor (NGF) ist ein Multikomponenten-Protein mit einem Molekulargewicht von ungefähr 130.000 Dalton. Es besteht aus den drei Untereinheiten α, β und γ. Seine Fähigkeit, das Wachstum von sensorischen Neuronen und sympathischen Neuronen zu bewirken, liegt jedoch lediglich in der β-Untereinheit. Der α-Untereinheit konnte bisher noch keine biologische Aktivität zugeordnet werden, während die γ-Untereinheit zumindest in einigen Zellspezies für die Prozessierung des β-NGF verantwortlich ist, welcher zunächst als langer Precursor (Vorläufer) synthetisiert wird. Der reife β-NGF besteht vermutlich aus 118 Aminosäuren. Er enthält 3 Disulfidbrücken und ist nicht glykosyliert. Um biologisch aktiv zu sein, muß er als Dimer vorliegen.

Nerve Growth Factor ist besonders interessant für die Behandlung von neurologischen Krankheiten wie z.B. der Alzheimer'schen Krankheit oder Parkinson'schen Krankheit. Es wurde daher bereits versucht, nachdem 1983 erstmals die cDNA-Sequenz beschrieben worden war (Ullrich et al., Nature Vol. 303 (1983), 821-825), das Protein auf gentechnologischem Wege herzustellen.

So wird in der EP-B 0 121 338 die DNA- und die Aminosäuresequenz von β-NGF sowie - hypothetisch - die Expression in E.coli beschrieben. Es wird in diesem europäischen Patent zwar ausgeführt, wie das Gen für β-NGF isoliert wurde und wie daraus die Sequenz erhalten wurde, jedoch wird für die direkte Expression in E.coli lediglich angegeben, welches Expressionssystem verwendet wurde. Es konnte hierbei jedoch kein aktives Protein erhalten werden.

In der EP-A 0 329 175 wird die Herstellung von aktivem humanem β-NGF unter Verwendung eines Fusionsgens beschrieben, in welchem dem β-NGF-Gen eine Erkennungssequenz für die Spaltung mit Thrombin vorangestellt ist. Die Fusion wird hierbei durch Thrombin nach der Expression des Fusionsproteins gespalten. Der Nachteil dieses Verfahrens liegt u.a. in der geringen Ausbeute an biologisch aktivem β-NGF.

In der EP-A 0 370 171 wird ein Verfahren zur Herstellung von aktivem β-NGF unter Verwendung eines Bakulovirus-Expressionssystems beschrieben. Dabei werden virusinfizierte Insektenzellen zur Expression verwendet. Die Kultivierung derartiger Zellen ist jedoch sehr aufwendig, wobei auch noch nicht deren Verwendbarkeit als wirtschaftlich interessante Produktionszellen gezeigt werden konnte.

In Bio.Chem.Bio.Phys. Research Commun. 171 (1990) 116-122 und EP-A 0 414 151 wird die Herstellung von aktivem β-NGF aus Eukaryonten beschrieben. Die Expression von rekombinanten Proteinen in eukaryontischen Zellen ist jedoch aufwendig und teuer.

In Gene 70 (1988), S. 57-65 wird die Expression von murinem β-NGF in E.coli beschrieben. Die Ausbeute an biologisch aktivem β-NGF war jedoch sehr gering.

In der EP-A 0 450 386 wird ein Verfahren zur Herstellung von β-NGF aus E.coli beschrieben. Das dabei erhaltene Produkt ist jedoch ein Gemisch aus β-NGF der natürlichen Sequenz und β-NGF mit einem N-terminalen Methioninrest im Verhältnis von ca. 85:15.

In Gene 83 (1989), S. 65-74 wird die Expression von humanem β-NGF in Hefe beschrieben. Auch hier ist die Expression nur gering, sowie die Aktivität des erhaltenen β-NGF nur sehr schwach.

Aufgabe der Erfindung war es daher, ein Verfahren bereitzustellen, welches die kostengünstige und einfache Herstellung von aktivem humanem β-NGF in Prokaryonten, insbesondere in E.coli ermöglicht, wobei eine hohe Expressionsrate und nach Naturierung ein Protein mit gleicher Aktivität wie ein aus natürlichen Quellen isolierter β-NGF erzielt werden sollte.

Gelöst wird die Aufgabe erfindungsgemäß durch ein Verfahren zur gentechnologischen Herstellung von biologisch aktivem β-NGF (Nerve Growth Factor β-subunit) durch Expression einer entsprechenden DNA-Sequenz in Prokaryonten als Wirtszellen, dadurch gekennzeichnet, daß die nach Expression erhaltenen unlöslichen Aggregate von inaktivem β-NGF durch
(1) Solubilisierung,
(2) gegebenenfalls Dialyse oder/und Derivatisierung des erhaltenen β-NGF mit der oxidierten Form einer Verbindung, deren reduzierte Form Sulfhydrylgruppen aufweist und deren oxidierte Form ein über eine Disulfidbrücke verbundenes Dimer der Verbindung ist, zum gemischten Disulfid, und
(3) Naturierung mit Hilfe eines Redoxsystems, bestehend aus einer Verbindung in oxidierter oder/und reduzierter Form, deren reduzierte Form Sulfhydrylgruppen aufweist und deren oxidierte Form ein über eine Disulfidbrücke verbundenes Dimer ist,
bei einer Temperatur zwischen 0 und 30°C, einem pH-Wert zwischen 8 und 10 sowie in Gegenwart von Tris-Base oder Tris-Salz und/oder Arginin in eine lösliche aktive Form von β-NGF überführt werden.

Mit Hilfe des erfindungsgemäßen Verfahrens wird es ermöglicht, β-NGF in hohen Ausbeuten in Prokaryonten herzustellen. In Prokaryonten fällt das Protein zunächst in Form von unlöslichen Aggregaten, sogenannten Inclusion Bodies (IBs) an und wird dann erst in die lösliche aktive Form überführt.

Die im erfindungsgemäßen Verfahren verwendete DNA-Sequenz entspricht der von Ullrich et al., Nature 303 (1983), 821-825 beschriebenen. Hierzu kann entweder in üblicher Weise das natürliche Gen isoliert und daraus die cDNA hergestellt werden. Auch eine Synthese einer DNA-Sequenz, bestehend aus den Exons des natürlichen Gens wäre möglich.

In einer bevorzugten Ausführungsform der Erfindung wird die Expression in E.coli als Wirtszellen und insbesondere im Stamm E.coli C600+ (DSM 5443) durchgeführt.

Die zur Expression einer DNA-Sequenz nötigen Faktoren sind dem Fachmann bekannt, hierzu gehören u.a. ein Promotor, ribosomale Bindungsstellen etc., welche auf den üblichen, für die Expression verwendeten Vektoren vorhanden sind. Im erfindungsgemäßen Verfahren ist es jedoch bevorzugt, einen induzierbaren Promotor zu verwenden und die Induktion dieses Promotors in der logarithmischen Wachstumsphase durchzuführen. Hierdurch wird es ermöglicht, eine besonders hohe Proteinausbeute zu erhalten, da nicht bereits in einer früheren Wachstumsphase die Expression des Fremdproteins zu einem Absterben der Wirtszellen führt. Beispiele für solche induzierbaren Promotoren sind der tac-Promotor, der durch Zugabe von IPTG induzierbar ist, der trp-Promotor oder der λP_{L}-Promotor.

Die Solubilisierung, Derivatisierung sowie Naturierung - zusammen auch Renaturierung genannt - von Proteinen, welche nach Expression in E.coli in Form von Inclusion Bodies anfallen, ist an sich bekannt und z.B. in der EP-A 0 219 874 beschrieben.

Die Solubilisierung - sie führt zu inaktivem, löslichem Protein - erfolgt mit Hilfe eines Denaturierungsmittels, vorzugsweise Guanidiniumhydrochlorid oder Harnstoff, wobei eine Konzentration des Denaturierungsmittels von 4 bis 6 mol/l (Guanidiniumhydrochlorid) und 5 bis 8 mol/l (Harnstoff) zu guten Ergebnissen führt.

Nach einer eventuellen Dialyse (diese ist nicht unbedingt nötig, jedoch wird sie vorzugsweise durchgeführt) kann dann gegebenenfalls eine Derivatisierung des Proteins zum gewünschten Disulfid erfolgen. In einer bevorzugten Ausführungsform wird im erfindungsgemäßen Verfahren die Derivatisierung mit GSSG, Cystamin oder Cystin durchgeführt. Vorzugsweise werden diese Verbindungen dem Inkubationsansatz in Mengen von bis zu 75 mmol/l zugegeben.

Nach der gegebenenfalls durchgeführten Derivatisierung (auch ohne die Derivatisierung ist die Renaturierung möglich) wird wieder vorzugsweise eine Dialyse zwischengeschaltet. Ebenso kann vor der Naturierung des solubilisierten Proteins eine Dialyse durchgeführt werden. Vorzugsweise wird zur Renaturierung bei Konzentrationen von Harnstoff unter 2 mol/l, vorzugsweise unter 0,5 mol/l gearbeitet und die Renaturierung analog der europäischen Patentanmeldung Nr. 91 919 945.5 in Gegenwart von Tris-Base oder Tris-Salz und/oder Arginin (analog EP-A 0 219 874) durchgeführt. Bevorzugt wird dabei im pH-Bereich zwischen 8 und 10 gearbeitet.

Die Naturierung, welche die solubilisierten bzw. derivatisierten Proteine in die letztendlich aktive lösliche Form überführt, wird erfindungsgemäß vorzugsweise mit oxidiertem Glutathion (GSSG) und/oder GSH, Cystin und/oder Cystein, Cystamin und/oder Cysteamin durchgeführt. Wurde keine Derivatisierung durchgeführt, ist es im erfindungsgemäßen Verfahren besonders bevorzugt, mit Cystamin, GSSG oder Cystin bzw. den Redoxsystemen Cystamin/Cysteamin, GSH/GSSG oder Cystin/Cystein zu naturieren. Wurde jedoch das Protein in die gemischten Disulfide überführt, also derivatisiert, wird vorzugsweise nur mit reduzierten Verbindungen naturiert. Die hierbei vorteilhaften Konzentrationen und weiteren Reaktionsbedingungen können leicht durch Orientierungsversuche festgestellt werden.

Besonders bevorzugt wird eine Pulsrenaturierung gemäß EP-A 0 241 022 durchgeführt.

Die Temperatur sollte bei der gesamten Renaturierung zweckmäßig zwischen 0 und 30°C im Reaktionsansatz liegen. Bei der Naturierung ist es jedoch bevorzugt, eine Temperatur von 2 bis 10°C einzuhalten, da die Ausbeute an aktivem β-NGF mit abnehmender Temperatur zunimmt. Vorzugsweise wird 2 bis 60 Stunden naturiert. Eine verlängerte Naturierung führt zu einer höheren Ausbeute an biologisch aktivem Protein.

Ein wesentlicher Vorteil des erfindungsgemäßen Verfahrens besteht darin, daß reproduzierbar einheitliche Proteine und nicht wie beispielsweise in EP-A 0 450 386 beschrieben Proteingemische (aus β-NGF mit und ohne N-terminalen Methioninrest) erhalten werden. Es wird rekombinanter β-NGF der natürlichen Sequenz erhalten, welcher leicht (z.B. über eine Mono-S-Säule) zu isolieren ist und dann 5% und weniger an Verunreinigungen durch modifizierten β-NGF oder β-NGF-Spaltprodukte enthält. Vorzugsweise enthält der rekombinante β-NGF 2% und weniger an diesen Verunreinigungen und ist völlig frei von anderen Säugerzellproteinen.

Ein weiterer Gegenstand der Erfindung ist somit ein rekombinant hergestellter, isolierter β-NGF der natürlichen Sequenz, welcher 5% und weniger, vorzugsweise 2% und weniger, an Verunreinigungen durch modifizierten β-NGF oder β-NGF-Spaltprodukten enthält und vorzugsweise frei von anderen Säugerzellproteinen ist. Das Protein wird erfindungsgemäß in hohen Ausbeuten erhalten und weist eine hohe Aktivität auf Die Dimerisierung zum natürlich wirksamen aktiven Protein erfolgt während der Renaturierung.

Für die erfindungsgemäßen Expressionsvektoren sind hierbei dem Fachmann bekannte Vektoren geeignet, wie z.B. Plasmide. Ein solcher Expressionsvektor enthält alle für die Expression in bestimmten Wirtsorganismen nötigen Elemente wie Promotoren, ribosomale Bindungsstellen, Terminationssequenzen etc. Erfindungsgemäß ist es bevorzugt, daß der Expressionsvektor einen induzierbaren Promotor enthält.

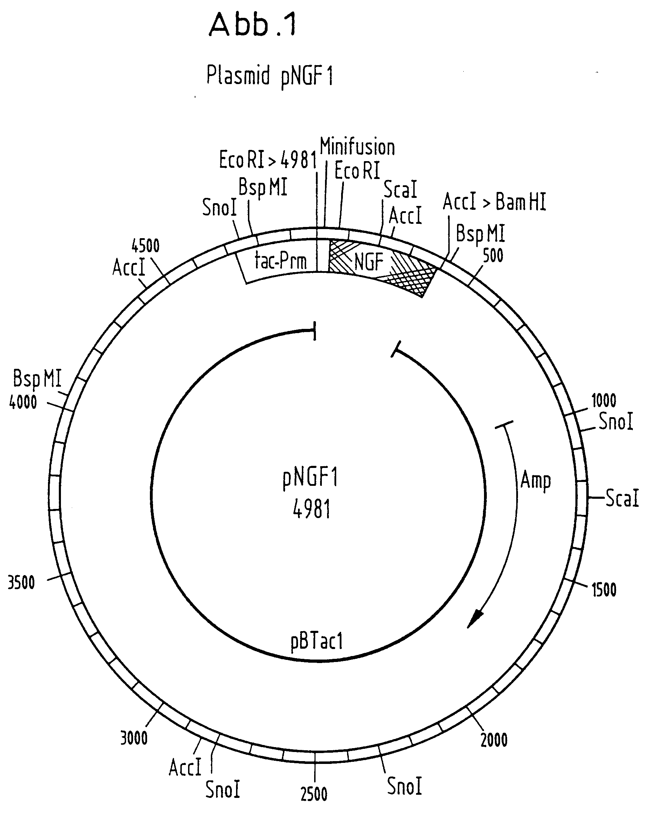

Ein solcher erfindungsgemäßer Expressionsvektor ist beispielsweise das Plasmid pNGF1, welches schematisch in Abb. 1 dargestellt ist.

N-terminal verlängerter und verkürzter β-NGF zeigen wie natürlicher β-NGF eine Wirkung auf Neuronen. Ein weiterer Gegenstand der Erfindung ist deshalb ihre Verwendung in einem Arzneimittel, ebenso wie die Verwendung des erfindungsgemäßen, rekombinant hergestellten β-NGF zu diesem Zweck.

Wiederum ein weiterer Gegenstand der Erfindung ist auch eine therapeutische Zusammensetzung, enthaltend neben üblichen Träger- und Hilfsstoffen einen erfindungsgemäßen rekombinant hergestellten oder rekombinanten N-terminal verlängerten oder verkürzten β-NGF als aktiven Inhaltsstoff.

Die in der vorliegenden Anmeldung genannten Plasmide und Mikroorganismen sind bei der Deutschen Sammlung von Mikroorganismen und Zellkulturen GmbH (DSM), Mascheroder Weg 1b, D-38124 Braunschweig, unter den folgenden Hinterlegungsnummern hinterlegt:
- E.coli C 600+: DSM 5443
- pUBS 520 in E.coli C 600+: DSM 6786
- pNGF1/pUBS 520 in E.coli C 600+: DSM 6785.

Die folgenden Beispiele erläutern in Verbindung mit dem Sequenzprotokoll und den Abbildungen die Erfindung weiter.
- Abb. 1: zeigt hierbei das erfindungsgemäße Plasmid pNGF1 in schematisierter Form.
- Abb. 2: zeigt die Primärstruktur des auf der DNA-Ebene codierten erfindungsgemäßen rekombinanten β-NGF mit den Sequenzen b (Ser-Tyr-Gln (SEQ ID NO:3)) und c (Arg-Thr-His-Arg-Ser-Lys-Arg (SEQ ID NO:2)).
- Abb. 3: zeigt die Abhängigkeit der Renaturierungsausbeute von der Guanidiniumhydrochloridkonzentration in der Renaturierungslösung. Die Renaturierung erfolgte durch eine 1/100 Verdünnung eines dialysierten Solubilisats mit 20 mg IB/ml in 0,7 mol/l Arginin, 1,0 mol/l Harnstoff, 0,1 mol/l Tris, 2 mmol/l EDTA, 5 mmol/l Cysteamin, 1 mmol/l Cystamin, pH 8,7, 4°C, mit unterschiedlichen Konzentrationen an Guanidiniumhydrochlorid.
- Abb. 4: zeigt die Abhängigkeit der Renaturierungsausbeute von der Proteinkonzentration in der Renaturierungslösung.
Die Renaturierung erfolgte durch eine 1/12,5 bis 1/1000-Verdünnung eines dialysierten Solubilisats mit 50 mg IB/ml in 0,7 mol/l Arginin, 2,0 mol/l Harnstoff, 0,1 mol/l Tris, 2 mmol/l EDTA, 5 mmol/l Cysteamin, 1 mmol/l Cystamin, pH 8,7. Guanidiniumhydrochlorid wurde in der Renaturierungslösung vor Zugabe des Solubilisats so aufgestockt, daß nach Zugabe des Solubilisats alle Lösungen 0,48 mol/l Guanidiniumhydrochlorid enthalten.

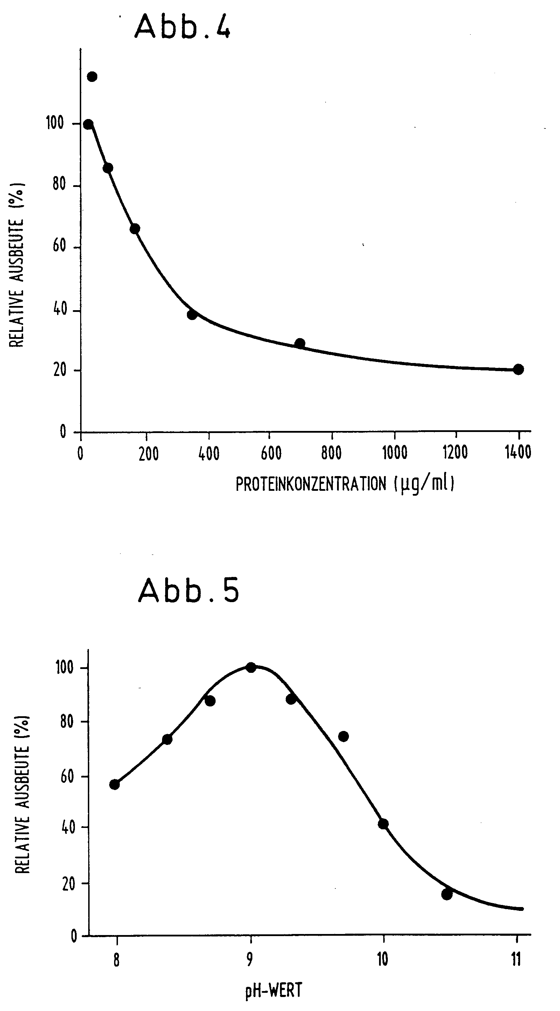

- Abb. 5: zeigt die Abhängigkeit der Renaturierungsausbeute vom pH-Wert der Renaturierungslösung. Die Renaturierung erfolgte durch 1/200-Verdünnung eines dialysierten Solubilisats mit 50 mg IB/ml in 0,7 mol/l Arginin, 2 mol/l Harnstoff, 0,1 mol/l Tris, 2 mmol/l EDTA, 5 mmol/l Cysteamin, 1 mmol/l Cystamin, pH 8,0 - 10,5, 4°C.

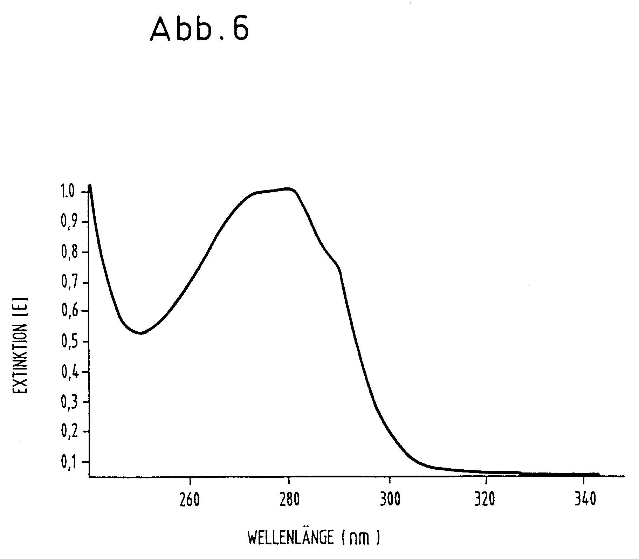

- Abb. 6: zeigt ein UV-Spektrum von rekombinantem modifiziertem (N-terminal verlängertem) β-NGF in 50 mmol/l Tris/HCl, 1 mmol/l EDTA, pH 7,5.

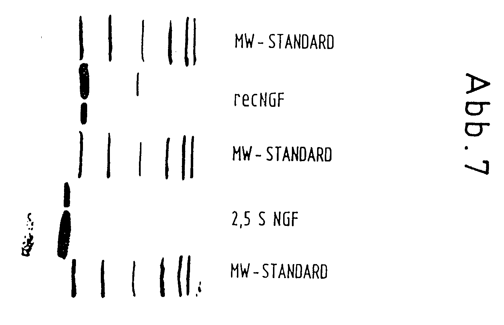

- Abb. 7: zeigt eine Comassie-Blue Färbung eines SDS-Gels von rekombinantem β-NGF im Vergleich mit 2,5 S-NGF aus Maus.

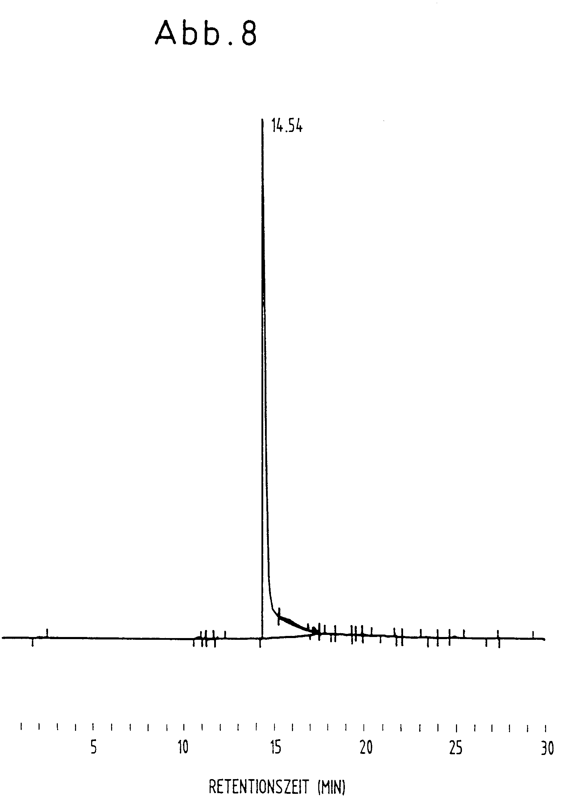

- Abb. 8: zeigt ein HPLC-Elutionsdiagramm von rekombinantem modifiziertem (N-terminal verlängertem) β-NGF.

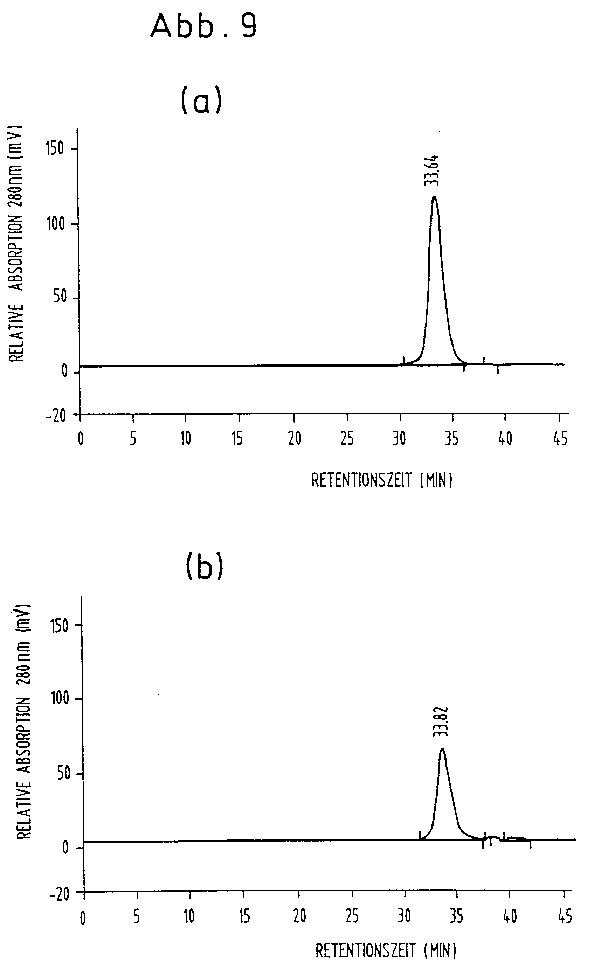

- Abb. 9: zeigt ein Superose 6-Elutionsdiagramm von rekombinantem (N-terminal verlängertem) β-NGF (Abb. 9a) und 2,5 S-NGF aus Maus (Abb. 9b).

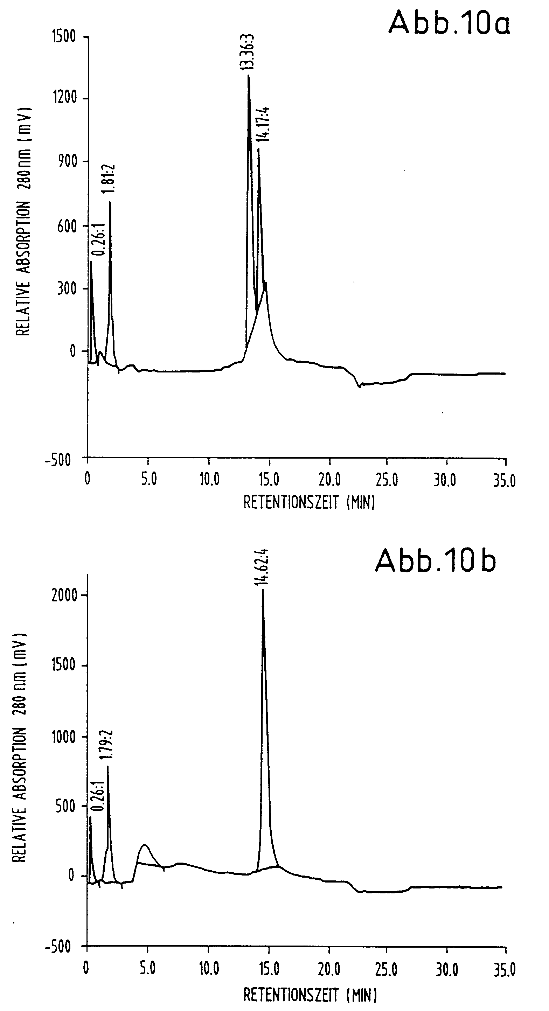

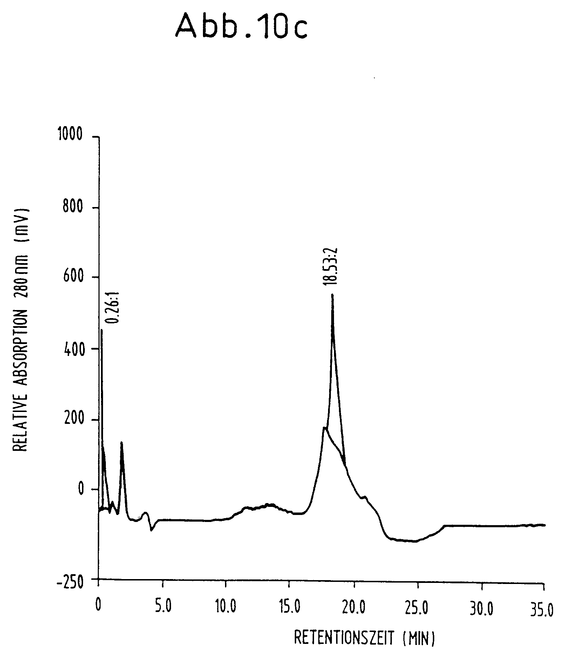

- Abb. 10: zeigt Mono S-Elutionsdiagramme von unverdautem rekombinantem β-NGF (Abb. 10c) bzw. Diagramme nach Verdau mit Trypsin (Abb. 10a) oder Endoproteinase Arg C (Abb. 10b).
- SEQ ID NO:1: zeigt die Aminosäuresequenz des rekombinanten β-NGF mit den N-terminalen Sequenzen SEQ ID NO:2 und 3.
- SEQ ID NO:2: zeigt einen Teil der Prosequenz des β-NGF Precursors.
- SEQ ID NO:3: zeigt einen Teil der N-terminalen Aminosäuresequenz von tPA.
- SEQ ID NO:4: zeigt einen Teil der N-terminalen Aminosäuresequenz von tPA.
- SEQ ID NO:5 und 6: zeigen Oligonukleotide zur Konstruktion von pNGF1.
- SEQ ID NO:7: zeigt die N-terminale Aminosäuresequenz von rec. NGF.
- SEQ ID NO:8 und 9: zeigen die N-terminale Aminosäuresequenz verschiedener N-terminal verkürzter β-NGF-Derivate.

### Beispiel 1

### Expression von rekombinantem β-NGF (rec β-NGF, β-NGF-Minifusion) in E.coli

### a) Konstruktion des Expressionsplasmids

Zur Herstellung des Expressionsplasmids wurden 3 Ansätze ligiert. Ansatz A stellt das etwa 4,6 kb große EcoRI/BamHI-Fragment des Plasmides pBTac1 dar. Das Plasmid pBTac1 wurde dazu mit den beiden Restriktionsenzymen EcoRI und BamHI behandelt. Der Restriktionsansatz wurde auf einem Agarose-Gel aufgetrennt. Das Fragment mit der Größe von etwa 4,6 kb wurde ausgeschnitten und die DNA aus dem Gel eluiert. Ansatz B wurde durch Restriktion des Plasmids pUC18-NGF (British Biotechnology, Code BBG26) hergestellt. Zur Restriktion wurden die beiden Enzyme BspMI und BamHI eingesetzt. Der Restriktionsansatz wurde gelelektrophoretisch aufgetrennt und ein BspMI/BamHI-Fragment von 370 bp präparativ gewonnen.

Ansatz C entstand durch das Annealing von zwei synthetisch hergestellten Oligonukleotiden mit folgender Sequenz:
Oligonukleotid 1: 5'-AATTCTTATGTCTTACCAAAGAACTCACCGTAGCAAGCGC-3' (SEQ ID NO:5)
Oligonukleotid 2: 5'-ATGAGCGCTTGCTACGGTGAGTTCTTTGGTAAGACATAAG-3' (SEQ ID NO:6)

In der Annealing-Reaktion wurden je 2 µg Oligonukleotide pro 10 µg eingesetzt; die Oligonukleotide wurden für 10 Minuten bei 68°C inkubiert und auf Raumtemperatur abgekühlt.

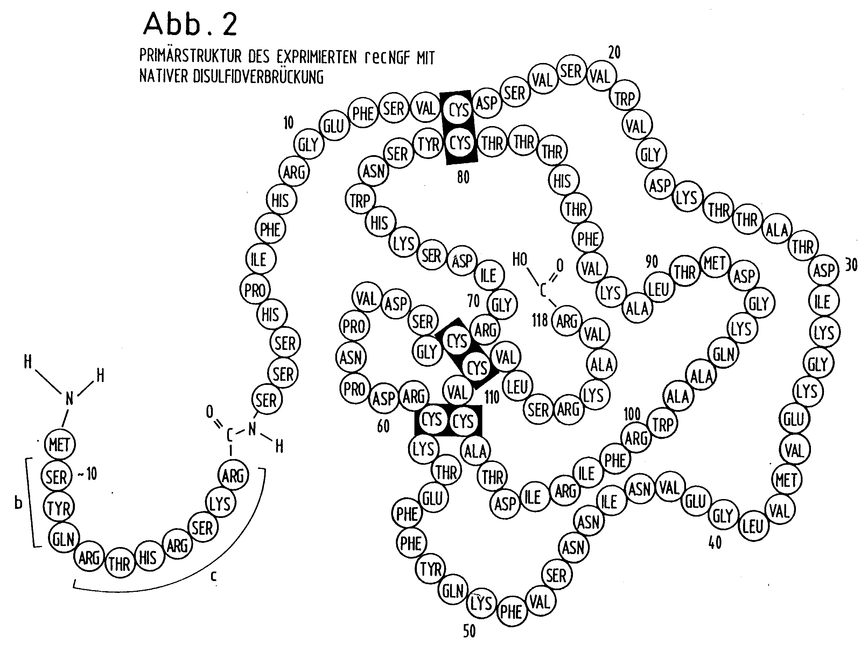

Die 3 Ansätze wurden nach bekannten Methoden ligiert und in E.coli C600+ (DSM 5443) zusammen mit dem Plasmid pUBS520 (beschrieben in Brinkmann et al., 1989, Gene 85, 109-114) transformiert. Durch Selektion auf Ampicillin und Kanamycin konnten die gewünschten Klone gefunden werden. Das erhaltene Plasmid pNGF1 ist in Abb. 1 beschrieben und unterscheidet sich vom Ausgangsplasmid pBTac1 u.a. dadurch, daß es eine zweite ScaI-Schnittstelle enthält. Die Aminosäuresequenz des codierten Proteins ist in Abb. 2 gezeigt.

### b) Expressionsstudien

Einer der erhaltenen Klone wurde im Hinblick auf Expression der β-NGF-Minifusion untersucht. Dazu wurden die Zellen bei 37°C bis zu einer optischen Dichte von 0,4 (gemessen bei 550 nm) wachsen gelassen. Die Expression des β-NGF-Fusionsproteins wurde durch die Zugabe von 5 mmol/l IPTG induziert. Die Zellen wurden für weitere 4 Stunden inkubiert und anschließend durch Zentrifugation konzentriert. Die Inclusion Bodies wurden nach bekannten Methoden (siehe Beispiel 2) präpariert und auf einem 15 % SDS-Polyacrylamidgel analysiert. Auf diese Weise konnte eine Expressionsstärke von mindestens 20 % bezogen auf das Gesamtprotein festgestellt werden.

### Beispiel 2

### Präparation von Inclusion Bodies (= IBs)

Zur Präparation der rec. β-NGF-enthaltenden IBs wurde der in Beispiel 1 beschriebene Expressionsstamm E.coli C600+ mit dem Plasmid pNGF1 in einem 10 l-Fermenter für 8 Stunden fermentiert. Die Induktion der Expression von β-NGF erfolgte durch die Zugabe von IPTG in der logarithmischen Wachstumsphase ca. 4 Stunden nach Fermentationsbeginn.

690 g Biomasse wurde nach 8 Stunden Fermentation mittels Zentrifugation geerntet. Die Biomasse wurde in 3,5 l 0,1 mol/l Tris/HCl pH 7 suspendiert und nach Zugabe von 0,3 g Lysozym 20 Minuten bei 0°C inkubiert. Der vollständige Zellaufschluß wurde anschließend mittels Hochdruckdispension mit 600 bar durchgeführt. Zur Aufschlußlösung wurde DNase ad 0,1 mg/ml und MgSO₄ ad 2 mmol/l zugegeben und die Lösung 30 Minuten bei 20°C inkubiert. Nach der DNase-Behandlung wurde die Lösung mit demselben Volumen 0,1 mol/l Tris/HCl, 6 % Triton-X-100, 1,5 mol/l NaCl, 60 mmol/l EDTA, pH 7,0 verdünnt und 20 Minuten im Eisbad inkubiert. Unlösliche Bestandteile (IBs) wurden anschließend durch Zentrifugation abgetrennt. Der Niederschlag wurde in 1 l 0,5 mol/l Guanidinium HCl (GdmCl), 20 mmol/l EDTA, pH 4,5 suspendiert. Nach 20 Minuten Inkubation bei 20°C wurden die IBs durch erneute Zentrifugation geerntet. Die folgende Resuspension des Niederschlags erfolgte in 1,5 l 0,1 mol/l Tris/HCl, 20 mmol/l EDTA, pH 6,5. Nach 30 Minuten Inkubation bei 20°C wurden die IBs durch eine weitere Zentrifugation im Niederschlag erhalten.

Zur Bestimmung des Anteils an β-NGF in den IBs wurden 500 mg IBs (Naßgewicht) mit einer Lösung aus 6 mol/l GdmCl, 0,1 mol/l Tris/HCl, 0,1 mol/l DTE, 5 mmol/l EDTA, pH 8,5 auf 10 ml aufgefüllt und 1 Stunde suspendiert. Der Proteingehalt der Lösung wurde mittels Proteinbestimmung nach Bradford (Bradford, M.M., Anal.Biochem. 1976, 72, 248) mit BSA als Eichprotein bestimmt. Der Anteil von β-NGF am Gesamtprotein in den gelösten IBs wurde nach Auftrennung der Proteine mittels SDS-Gelelektrophorese durch densitometrische Vermessung der Probenbahn ermittelt. Die in den aus 10 l Fermentationsbrühe isolierten IBs enthielten 6,0 g β-NGF.

### Beispiel 3

### Solubilisierung und Derivatisierung von rec. β-NGF

### a) Herstellung von Solubilisat:

IBs wurden in einer Lösung aus 6 mol/l GdmCl, 0,1 mol/l DTE, 5 mmol/l EDTA, 0,1 mol/l Tris/HCl, pH 8,5 zu einer Konzentration von 5 bis 50 g IB/1 l suspendiert und 1 Stunde bei 25°C gerührt.

Teilweise wurde unmittelbar das so erzeugte Solubilisat für die anschließende Naturierung verwendet.

### b) Herstellung von dialysiertem Solubilisat:

Alternativ wurde der pH-Wert des Solubilisats mit HCl (25 %) auf pH 3 eingestellt und die saure Lösung mehrfach gegen 6 mol/l GdmCl, 5 mmol/l EDTA, pH 3 dialysiert. Dieses Dialysat wurde sowohl zur Renaturierung als auch zur anschließenden Derivatisierung verwendet.

### c) Herstellung von Derivaten:

Zur Herstellung der Derivate (gemischte Disulfide) wurde der pH-Wert des dialysierten Solubilisats durch Zugabe von 5 N NaOH auf pH 8,5 gestellt. Anschließend wurde mit 1 Volumen einer Lösung aus 6 mol/l GdmCl, 0,2 mol/l Tris HCl, 5 mmol/l EDTA pH 8,5 verdünnt und oxidiertes Glutathion (GSSG), Cystamin oder Cystin in fester Form bis zu einer Konzentration von 75 mmol/l zugegeben und 1 Stunde bei 20°C inkubiert. Im Falle von Cystin erfolgte die Inkubation bei pH 9,5 anstelle von pH 8,5. Anschließend wurde der pH-Wert der Lösung mit 25 % HCl auf pH 3 eingestellt und mehrmals gegen 6 mol/l GdmCl, 1 mmol/l EDTA, pH 3,0 dialysiert.

### Beispiel 4

### Naturierung von rec. β-NGF

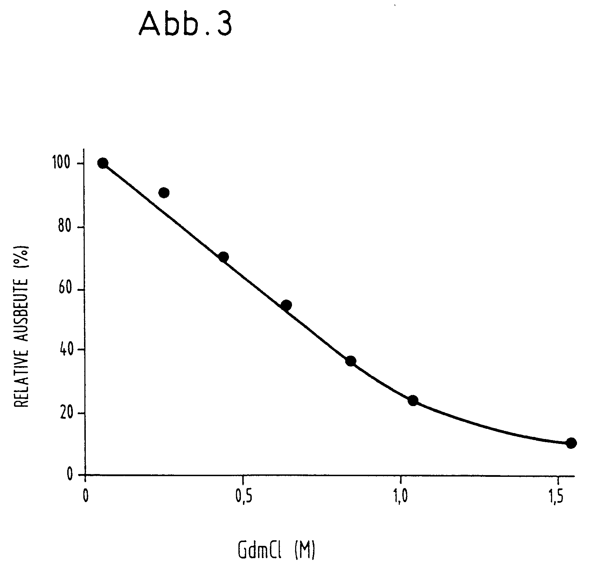

Zur Darstellung von biologisch aktivem β-NGF aus den in Beispiel 3 hergestellten Solubilisaten und Derivaten wurden die β-NGF in inaktiver, löslicher Form enthaltenden Lösungen 12,5- bis 1000-fach in unterschiedliche Renaturierungspuffer verdünnt. Zur Ermittlung der optimalen Renaturierungsbedingungen wurden die Proteinkonzentration, der pH-Wert, die Temperatur sowie die Konzentration an Arginin, Tris/HCl, Harnstoff GdmCl, oxidiertem und reduziertem Glutathion (= GSSG bzw. GSH), Cystin und Cystein und Cystamin und Cysteamin variiert. Die Ergebnisse sind in Tabellen 1 bis 3 und Abb. 3 bis 5 dargestellt.

Zur Bestimmung der Konzentration an renaturiertem, biologisch aktivem β-NGF in den Renaturierungslösungen wurde die Stimulierbarkeit durch β-NGF von sensorischen Neuronen aus dissoziierten Dorsalwurzelganglien von Hühnerembryonen zum Austreiben von Dendriten (= DRG-Test = dorsal root ganglion assay, Levi-Montalcini, R., Meyer H. und Hamburger, V. 1954, Cancer Res., 14, 49-57; Varon, S., Nomura, J., Perez-Polo, J.R. und Shooter, E.M., 1972, Meth. in Neurochemistry 3, 203-229; Europ. Patentanmeldung EP 0 335 673 A2, S14-15, Beispiel C), sowie ein Sandwich-Enzym-Immunoassay (β-NGF-ELISA) verwendet.

Für den DRG-Test wurden die Renaturierungslösungen nach 24 Stunden Inkubationszeit gegen 50 mmol/l Tris/HCl pH 7,5 dialysiert und anschließend durch Zentrifugation geklärt. In den Überständen wurden je 100 µl pur oder nach Verdünnung mit Medium (F14-Medium; Coon, M.G. und Weiß, M.C., 1969, Proc.Natl.Acad.Sci. USA 62, 852-859) in 24-well (Vertiefung) Zellkulturplatten (Fa. Costar) mit 750 µl Medium und 150 µl Zellsuspension der dissoziierten Ganglien gemischt und 48 Stunden bei 37°C inkubiert. Als Maß für die biologische Aktivität wurde die Anzahl der Zellen mit ausgebildeten Dendriten quantifiziert. Als Referenz wurde eine Lösung bekannter Konzentration von 2,5 S β-NGF aus Submaxillaris-Drüsen der Maus (Fa. Boehringer Mannheim) verwendet.

Für den β-NGF-ELISA wurden für den Vergleich der Ergebnisse mit dem DRG-Test ebenfalls gegen 50 mmol/l Tris/HCl pH 7,5 dialysierte Renaturierungslösungen verwendet. Ansonsten wurden die Renaturietungslösungen umittelbar nach Klärung mittels Zentritugation eingesetzt.

Zum Nachweis von renaturiertem β-NGF mittels ELISA wurde eine 96-well Mikrotiterplatte (Fa. Nunc-Immuno; Typ Maxisorp F96) mit einem monoklonalen Antikörper gegen Maus 2,5 S NGF (Fa. Boehringer Mannheim GmbH) beschichtet, indem pro well mit 0,1 ml Antikörperlösung aus 0,3 µg Antikörper/ml 50 mmol/l Na₂CO₃, 0,1 % Na-azid, pH 9,6 für 16 Stunden bei 4°C inkubiert wurde. Nach dreimaligem Waschen mit 0,2 ml 50 mmol/l Tris/HCl, 0,2 mol/l NaCl, 10 mmol/l CaCl2, 0,2 % Triton X-100, 0,1 % Na-azid, pH 7,0 pro well wurden je 100 µl β-NGF-enthaltende Renaturierungslösungen sowie deren Verdünnungen in Waschpuffer mit zusätzlich 1 % BSA gefüllt. Zum Vergleich mit dem Zelltest wurden die entsprechenden dialysierten und zentrifugierten Lösungen verwendet, ansonsten wurden die Renaturierungslösungen unmittelbar nach Klärung mittels Zentrifugation eingesetzt. Als Referenz wurde eine Verdünnungsreihe von 2,5 S-NGF aus Submaxillaris-Drüsen der Maus (Fa. Boehringer Mannheim) im Konzentrationsbereich 0,1 µg/ml bis 20 pg/ml je Platte mitgeführt. Nach 2 Stunden Inkubation bei 37°C wurde die Lösung durch dreimaliges Waschen entfernt. Anschließend wurde in jedes well 0,1 mol einer Lösung von β-Galactosidase-konjugierten monoklonalen Antikörper gegen Maus 2,5 S NGF (Fa. Boehringer Mannheim; 0,4 U/ml in Waschpuffer mit 1 % BSA) gegeben und 4 Stunden bei 37°C inkubiert.

Nach einem erneuten Waschzyklus wurde jedes well mit 0,2 ml einer Lösung von 40 mg Chlorphenolrot-β-D-galactopyranosid in 20 ml 100 mmol/l Hepes, 150 mmol/l NaCl, 2 mmol/l MgCl2, 0,1 % Na-azid, 1 % BSA pH 7 gefüllt. Je nach Farbentwicklung wurde nach 10 bis 60 Minuten die Absorption der Proben mit einem ELISA-Reader (z.B. Typ EAR 400 AT von Fa. SLT-Labinstruments, Austria) gemessen. Die β-NGF-Konzentration in Renaturierungslösungen wurde anhand der Eichkurve mit 2,5 S NGF aus Maus berechnet.

Sofern nicht anders vermerkt, wurden die Renaturierungslösungen nach einer Inkubationszeit von 24 Stunden analysiert.

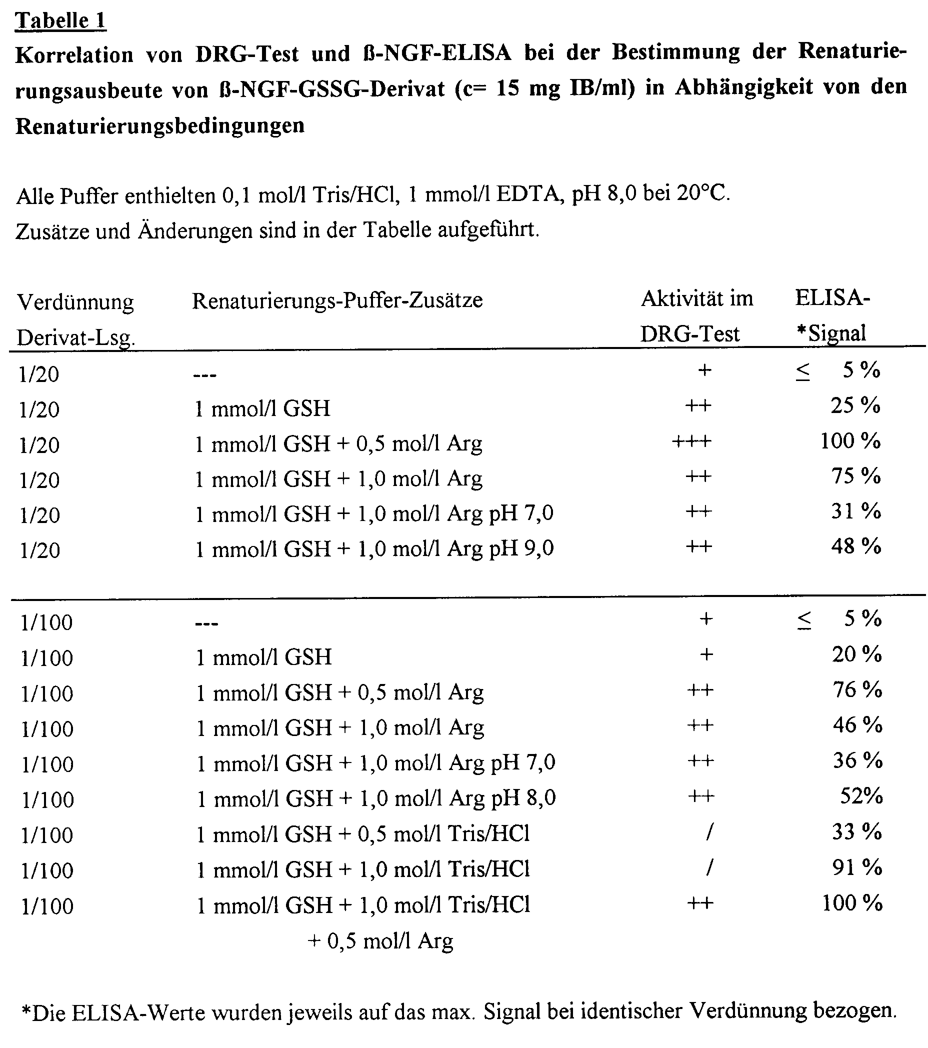

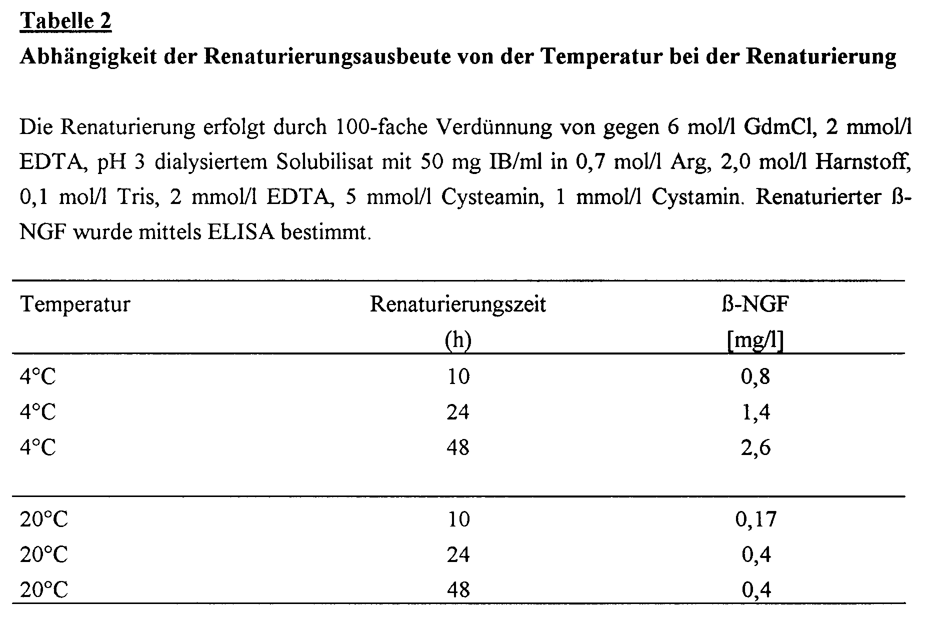

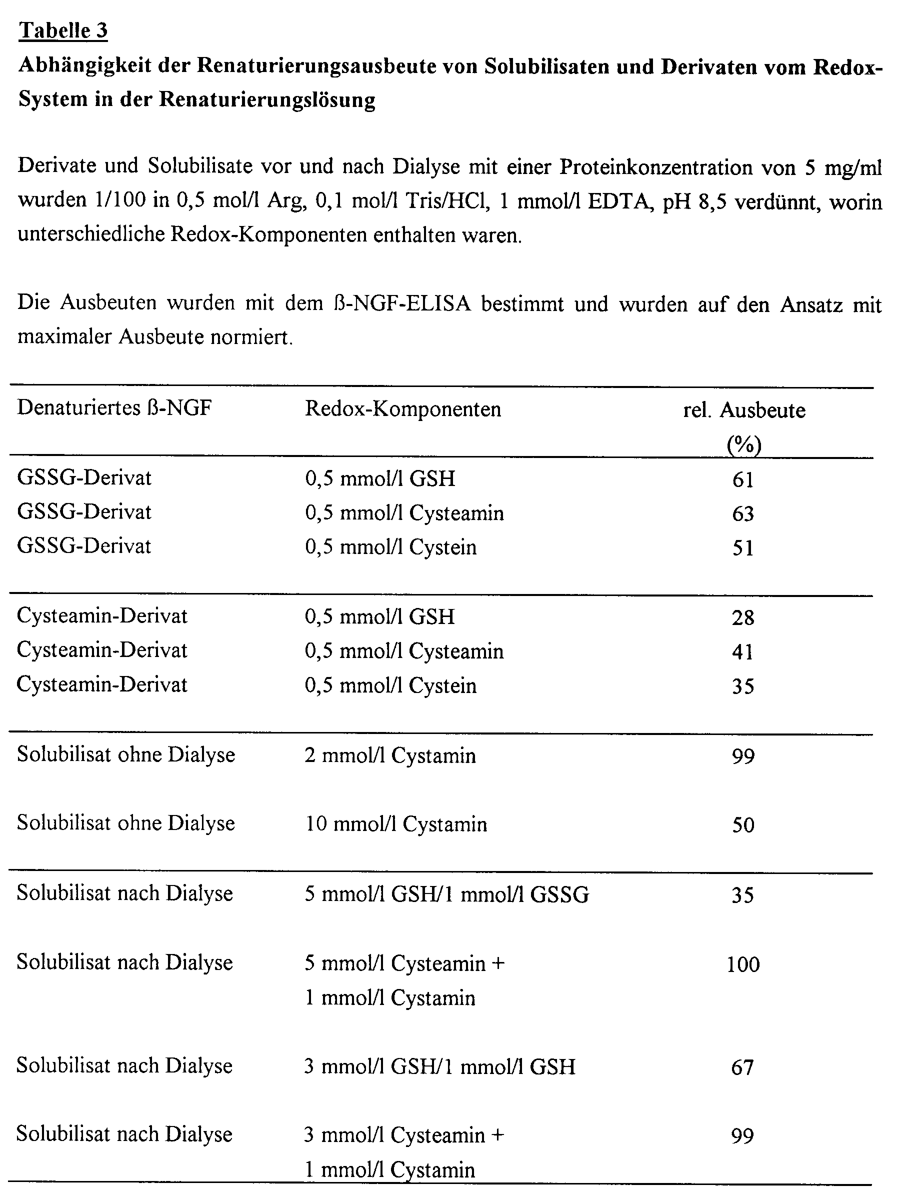

### Beispiel 5

### Pulsrenaturierung von rec. β-NGF

20 g IB-Material wurde in 400 ml 6 mol/l GdmCl, 0,1 mol/l Tris/HCl, 0,1 mol/l DTE, 2 mmol/l EDTA pH 8,5 gelöst und 1 h bei 20°C inkubiert. Anschließend wurde der pH-Wert der Lösung auf pH 3 mit 25 % HCl eingestellt und gegen 3 x 10 l 6 mol/l GdmCl, 2 mmol/l EDTA, pH 3 bei 4°C dialysiert. Je 100 ml Dialysat wurden im Abstand von 12 bis 16 Stunden innerhalb von 30 Minuten zu 20 l 0,7 mol/l Arg, 2 mol/l Harnstoff, 0,1 mol/l Tris, 2 mmol/l EDTA, 5 mmol/l Cysteamin, 1 mmol/l Cystamin, pH 9,1, 4°C zugepumpt.

**Tabelle 4**

| **Mit ELISA bestimmte β-NGF-Konzentration im Renaturierungsansatz nach Zugabe unterschiedlicher Volumina an dialysiertem Solubilisat** | | |
|---|---|---|
| Pulsanzahl | Zugegebenes Solubilisat | β-NGF-Konz. (mg/l) |
| 1 | 100 ml | 3,5 |
| 2 | 200 ml | 5,3 |
| 4 | 400 ml | 8,5 |

### Beispiel 6

### Reinigung von biologisch aktivem rec. β-NGF aus der Renaturierungslösung

Zu der in Beispiel 5 hergestellten Renaturierungslösung wurde festes Ammoniumsulfat portionsweise bis zu einer Sättigung von 20 % zugegeben und 1 h auf Eis gerührt. Der pH-Wert der Lösung wurde anschließend mit 25 %iger HCl auf pH 3 eingestellt. Nach 30 Minuten Inkubation wurde mit 7 N NaOH auf pH 8 titriert. Trübungen wurden entweder durch Zentrifugation (1 h mit 20 000 g bei 4°C) oder Filtration durch eine geeignete Membran mit geringer Proteinadsorption (z.B. Evaflux 4 A Hohlfaserpatrone der Fa. Kuraray Co. Ltd., Japan) entfernt. Die klare β-NGF enthaltende Lösung wurde dann auf eine TSK-Butyl-Säule (Fa. Pharmacia) aufgetragen, die vorher mit 50 mmol/l Tris/HCl, 1 mmol/l EDTA, 20 % Sättigung an (NH₄)₂SO₄, pH 7,5 äquilibriert wurde. Nach Beendigung des Probenauftrags wurde mit 50 mmol/l Tris/HCl, 1 mmol/l EDTA pH 7,5 bis zum Erreichen der Schreibergrundlinie gespült. Die Elution erfolgte mit 50 % Ethylenglykol in 50 mmol/l Na-Acetat, 1 mmol/l EDTA, pH 4,5. Eluierende Fraktionen wurden mit dem ELISA (s. Beispiel 4) auf den Gehalt an β-NGF getestet. β-NGF enthaltende Fraktionen wurden vereint und auf eine Sephacryl S200-Säule (Fa. Pharmacia, Schweden) aufgetragen, die mit 0,5 mol/l NaCl, 50 mmol/l Na-Phosphat, 10 % Glycerin, 1 mmol/l EDTA, pH 6,0 äquilibriert war. Die Säule wurde mit demselben Puffer eluiert.

β-NGF enthaltende Fraktionen im Eluat wurden mit H₂O auf eine Leitfähigkeit von 15 mS verdünnt und auf eine mit 20 mmol/l Natriumphosphat, 1 mmol/l EDTA, pH 6,0 äquilibrierte S-Sepharose-Säule (Fa. Pharmacia) aufgetragen. Anschließend wurde mit dem Äquilibrierungspuffer gespült. Die Elution erfolgte mit einem Gradienten bis 1,2 mol/l NaCl in obigem Puffer. β-NGF enthaltende Fraktionen wurden vereinigt und, sofern für weitere Analysen erforderlich, durch Dialyse umgepuffert.

**Tabelle 5**

| **Ausbeuten der Reinigung von rec. β-NGF aus 20 l Renaturierungslösung nach Bestimmung mit ELISA** | | |
|---|---|---|
| Reinigungsschritt | β-NGF (mg) | Ausbeute (%) |
| Renaturierungslösung | 170 | 100 |
| vor TSK-Butyl | 143 | 84 |
| nach TSK-Butyl | 92 | 54 |
| nach S200 | 80 | 47 |
| nach S-Sepharose | 61 | 36 |

### Beispiel 7

### Charakterisierung des gereinigten rec. β-NGF

### a) Konzentrationsbestimmung mit UV-Spektralphotometrie:

Zur Bestimmung der Konzentration an β-NGF in den gereinigten Proben wurde das UV-Spektrum von 240 bis 340 nm von gegen 50 mmol/l Tris/HCl, 1 mmol/l EDTA, pH 7,5 dialysierten Proben mit einem UV/Vis-Spektralphotometer (z.B. Diodenarray-Spektralphotometer Typ HP 8452 A der Fa. Hewlett-Packard, USA) gemessen. Die β-NGF-Konzentration in der Lösung wurde aus der Absorption der Probe bei 280 nm berechnet. Hierzu wurde ein theoretischer molarer Extinktionskoeffizient von 21200 ± 100 M⁻¹ cm⁻¹ verwendet, der sich aus dem Gehalt an bei dieser Wellenlänge absorbierenden Aminosäuren ergibt (Wetlaufer, D.B., 1962, Adv. in Protein Chemistry, Vol. 17, 375-383; Gill, S.C. und von Hippel, P.H., 1989, Anal.Biochem. 182, 319-326). Als Molekulargewicht wurden 14000 D verwendet. Die mittels Absorption errechneten Werte ergaben eine um ca. 50 % niedrigere β-NGF-Konzentration als mit ELISA bestimmt wurde. Ursache könnte die geringere Reinheit des als Referenz verwendeten 2,5 S-NGF (aus Maus) sein, weshalb die mit ELISA ermittelten Werte für rec. β-NGF zu hoch sind.

### b) Reinheitsanalyse und Molekulargewichtsbestimmung mit SDS-Polyacrylamidgelelektrophorese:

Es wurden 17 %ige Gele in einem Midget-System (Fa. Pharmacia, Schweden) verwendet. Die Proben enthielten jeweils 10 mmol/l DTE sowie nach Absorption bei 280 nm berechnet 6 µg bzw. 1,5 µg rec. β-NGF sowie 8,4 µg bzw. 2,1 µg 2,5 S-NGF aus Submaxillaris-Drüsen der Maus. rec. β-NGF hat wie erwartet mit ca. 15 kD ein etwas höheres Molekulargewicht als 2,5 S-NGF mit ca. 12,5 kD. Die Bande im Molekulargewichtsbereich des dimeren rec. β-NGF tritt in Abhängigkeit von der Probenvorbereitung und vom Probenalter in unterschiedlicher Intensität aufund kann auf die Bildung Disulfid-verbrückter Moleküle bei der Probenvorbereitung zurückgeführt werden. Die densitometrische Vermessung einer Gelspur (Ultroscan XL Laser Densitometer, Fa. Pharmacia, Schweden) mit rec β-NGF ergab 93% Monomer und 7 % Dimer.

### c) Reinheitsanalyse mit RP-C18-HPLC:

20 µg rec. ß-NGF wurden auf eine mit 0,1 % TFA äquilibrierte Vydac 300, C18, 5 µ, 125 x 4 mm-Säule aufgetragen und bei einer Flußrate von 1 ml/Minute mit einem gestuften Gradienten von 0 bis 40 % B (B = Acetonitril/0,1% TFA) in 20 Minuten und 40 - 100 % B von 20 bis 25 Minuten eluiert. Zur Detektion wurde die Fluoreszenzemission bei 340 nm nach Anregung bei 280 nm (Merck/Hitachi F-1050 Fluorescence Spektrophotometer) benutzt. Die Integration des Elutionsdiagramms ergab eine Reinheit von 92 %. Identische Ergebnisse wurden mit UV-Detektion bei 280 nm erhalten. Die Identität des Elutionspeaks mit β-NGF wurde nach Sammeln und Einengen mittels Speed Vac durch ELISA und N-terminale Sequenzierung überprüft.

### d) Reinheitsanalyse von rec β-NGF mit SEC-HPLC:

30 µl einer gereinigten rec β-NGF-Lösung mit einer Absorption bei 280 nm von 1,3 wurden auf eine Superose 6-Säule Typ HR 10/30 (Fa. Pharmacia, Schweden) aufgetragen und mit 0,7 mol/l Arginin, 0,1 mol/l Tris, pH 8,5 bei einem Fluß von 0,5 ml/Minute eluiert. Es wurde die Absorption bei 280 nm detektiert. Abb. 9 zeigt das Elutionsdiagramm im Vergleich zu einer Probe des 2,5 S-NGF aus Maus.

### e) N-terminale Sequenzanalyse:

Für die N-terminale Sequenzanalyse wurde das in Beispiel 6c mittels RP-HPLC isolierte und anschließend konzentrierte β-NGF verwendet. Die N-terminale Sequenz wurde mit einem Gasphasen-Protein-Sequenzanalysator (Modell 477 A, Fa. Applied Biosystems, USA) durchgeführt. Es wurde folgende Sequenz mit einer Signalstärke von ca. 400 pmol pro PTH-Aminosäure gefunden:
H₂N-Ser-Tyr-Gln-Arg-Thr-His-Arg-Ser (SEQ ID NO:7)

Die N-terminale Aminosäure Methionin wird in E.coli offenbar bereits abgespalten.

### f) Biologische Aktivität des rec. β-NGF und deren Inhibition durch Anti-NGF-β-Antikörper:

Der DRG-Test zur Bestimmung der biologischen Aktivität von β-NGF wurde wie in Beispiel 4 beschrieben durchgeführt. Als Proben wurde das isolierte rec. β-NGF nach Proteinbestimmung mittels Absorption bei 280 nm, sowie 2,5 S-NGF aus Submaxillaris-Drüsen der Maus eingesetzt. Die Proben wurden mit Medium auf 200, 20 und 2 ng/ml verdünnt, wovon je 100 µl mit 750 µl Medium und 150 µl Zellsuspension gemischt und inkubiert wurden. Zur Inhibition wurde der Zelltest mit einem Medium durchgeführt, das einen monoklonalen Anti-β-(2,5 S)-NGF-Antikörper enthielt, so daß die Antikörperkonzentration im Testansatz 200 ng/ml betrug. Die Ergebnisse sind in Tabelle 6 gezeigt.

**Tabelle 6**

| **Biologische Aktivität von rec. β-NGF und deren Inhibition durch Anti-NGF-β-Antikörper** | | | |
|---|---|---|---|
| β-NGF-Spezies | β-NGF-Konz. (ng/ml) | Antikörper (ng/ml) | biologische Aktivität |
| rec. β-NGF | 20,0 | --- | +++ |
| rec. β-NGF | 2,0 | --- | +++ |
| rec. β-NGF | 0,2 | --- | ++ |
| rec. β-NGF | 20,0 | 200 | +/- |
| rec. β-NGF | 2,0 | 200 | - |
| rec. β-NGF | 0,2 | 200 | - |
| 2,5 S β-NGF | 20,0 | --- | +++ |
| 2,5 S β-NGF | 2,0 | --- | ++ |
| 2,5 S β-NGF | 0,2 | --- | ++ |
| 2,5 S β-NGF | 20,0 | 200 | + |
| 2,5 S β-NGF | 2,0 | 200 | - |
| 2,5 S β-NGF | 0,2 | 200 | - |

### Beispiel 8

### Herstellung und Charakterisierung N-terminal verkürzter rec. β-NGF-Moleküle

Zur Herstellung N-terminal verkürzter rec β-NGF-Moleküle wurde eine gereinigte Probe gegen 50 mM Tris/HCl, pH 7,7 dialysiert. Die Konzentration wurde mittels der Absorption der Probe bei 280 nm (siehe Beispiel 7a) mit 0,7 mg/ml bestimmt.

Verkürzte rec. β-NGF-Moleküle wurden durch Verdau mit den Endoproteinasen Trypsin und Arg C in folgenden Verdauansätzen hergestellt.
a) rec. NGF + Trypsin: 22°C
   - 200 µl: rec. β-NGF-Dialysat
   - + 60 µl: Trypsin sequence grade-Lösung
   (100 µg/200 µl 0,1 mol/l Tris, pH 8,0)
b) rec. NGF + Endoproteinase Arg C: 37°C
   - 200 µl: rec. β-NGF-Dialysat
   - + 20 µl: 50 mmol/l CaCl₂, 50 mmol/l DTE
   100 mmol/l Tris/HCl, pH 7,6
   - + 40 µl: Endoproteinase Arg C-Lösung
   (20 µg in 40 µl 250 mmol/l Tris/HCl, 25 mmol/l DTE, 2,5 mmol/l EDTA, 5 mmol/l CaCl₂ pH 7,8)
c) rec. NGF ohne Protease: 22°C
   - 200 µl: rec. β-NGF-Dialysat
   - + 60 µl: 50 mmol/l Tris/HCl pH 7,7

Weiterhin wurden analoge Kontroll-Lösungen mit den Proteasen angesetzt, die anstelle von rec. β-NGF den Dialysepuffer enthielten. Die Ansätze wurden 5 Stunden bei den entsprechenden Temperaturen inkubiert. Anschließend wurden je 200 µl der Ansätze auf eine mit 20 mmol/l Natriumphosphat pH 6,0 äquilibrierte Mono S-Säule Typ HR 5/5 (Fa. Pharmacia) mit einer Flußrate von 1 ml/Minute aufgetragen und mit einem linearen Gradienten von 0 - 1,2 mol/l NaCl eluiert. Es wurde die Absorption bei 280 nm detektiert. Die Kontrollansätze nur mit Proteasen ergaben, daß diese innerhalb der ersten 10 Minuten der Chromatographie eluieren. β-NGF-enthaltende Fraktionen eluieren je nach Verkürzung erst mit einer Retentionszeit von mindestens 10 Minuten. Die Elutionsdiagramme der Chromatographien von rec. β-NGF-enthaltenden Verdauansätzen sind in Abb. 10 gezeigt. Elutionspeaks mit Retentionszeiten von 13 - 19 Minuten wurden jeweils gesammelt. Die beiden peaks im Verdauansatz mit Trypsin wurden getrennt aufgefangen.

Die β-NGF-Konzentrationen in den Fraktionen der aufgefangenen peaks wurden mittels der Absorption der Proben bei 280 nm ermittelt. Je 1 nmol rec β-NGF-enthaltende Lösungen wurden mittels RP-C18-HPLC konzentriert und die N-terminale Sequenz durch Gasphasensequenzierung bestimmt. Zusätzlich wurden Proben der gesammelten Fraktionen auf β-NGF-Konzentrationen von 20, 10 und 5 ng/ml in den DRG-Test verdünnt, um die biologische Aktivität der verkürzten β-NGF-Moleküle zu bestimmen. Die Inhibition der biologischen Aktivität mit einem anti-β-NGF-Antikörper erfolgte wie in Beispiel 7f beschrieben.

Die Ergebnisse der Sequenzanalyse und des Zelltests sind in Tabellen 7 und 8 zusammengefaßt.

**Tabelle 7**

| **N-terminale Sequenzen der proteolytisch verkürzten β-NGF-Moleküle** | | | |
|---|---|---|---|
| verwendete Protease | Retent.zeit β-NGF auf MonoS | N-terminale Sequenz | Position * 1. A.S. |
| --- | 18,5 Minuten | Ser Tyr Gln Arg Thr His Arg Ser (SEQ ID NO:7) | -9 |
| Trypsin | 13,4 Minuten | Gly Glu Phe Ser Val Cys Asp Ser (SEQ ID NO:8) | + 10 |
| Trypsin | 14,2 Minuten | Ser Ser Ser His Pro Ile Phe His (SEQ ID NO:9) | + 1 |
| Arg C | 14,6 Minuten | Ser Ser Ser His Pro Ile Phe His (SEQ ID NO:9) | + 1 |

| | | | |
|---|---|---|---|
| *Die angegebenen Positionen der jeweils ersten identifizierten Aminosäure (= A.S.) beziehen sich auf die Numerierung der Aminosäuren in Abb. 2. | | | |

**Tabelle 8**

| **Biologische Aktivität von N-terminal verkürzten β-NGF im DRG-Test** | | | |
|---|---|---|---|
| β-NGF-Spezies | β-NGF-Konz. (ng/ml) | Antikörper (ng/ml) | biologische Aktivität |
| rec β-NGF (unverdaut) | 20 | --- | +++ |
| | 10 | --- | +++ |
| | 20 | 200 | - |
| Trypsinverdau Peak 13,4 Minuten | 20 | --- | +++ |
| | 10 | --- | +++ |
| | 20 | 200 | - |
| Trypsinverdau Peak 14,2 Minuten | 20 | --- | +++ |
| | 10 | --- | +++ |
| | 20 | 200 | - |
| Arg C-Verdau | 20 | --- | +++ |
| | 10 | --- | +++ |
| | 20 | 200 | - |

## Patentansprüche

1. Verfahren zur gentechnologischen Herstellung von biologisch aktivem β-NGF (Nerve Growth Factor) durch Expression einer entsprechenden DNA-Sequenz in Prokaryonten als Wirtszellen, dadurch gekennzeichnet, daß man die nach Expression erhaltenen unlöslichen Aggregate von inaktivem β-NGF durch
(1) Solubilisierung,
(2) gegebenenfalls Dialyse oder/und Derivatisierung des erhaltenen β-NGF mit der oxidierten Form einer Verbindung, deren reduzierte Form Sulfhydrylgruppen aufweist und deren oxidierte Form ein über eine Disulfidbrücke verbundenes Dimer der Verbindung ist, zum gemischten Disulfid, und
(3) Naturierung mit Hilfe eines Redoxsystems, bestehend aus einer Verbindung in oxidierter oder/und reduzierter Form, deren reduzierte Form Sulfhydrylgruppen aufweist und deren oxidierte Form ein über eine Disulfidbrücke verbundenes Dimer ist,
bei einer Temperatur zwischen 0 und 30°C, einem pH-Wert zwischen 8 und 10 sowie in Gegenwart von Tris-Base oder Tris-Salz und/oder Arginin in eine lösliche aktive Form von β-NGF überführt.

2. Verfahren nach Anspruch 1, dadurch gekennzeichnet, daß man die Solubilisierung mit Hilfe eines Denaturierungsmittels, insbesondere Guanidiniumhydrochlorid oder Harnstoff durchführt.

3. Verfahren nach einem der vorhergehenden Ansprüche, dadurch gekennzeichnet, daß man für die Derivatisierung oxidiertes Glutathion (GSSG), Cystin oder Cystamin verwendet.

4. Verfahren nach einem der vorhergehenden Ansprüche, dadurch gekennzeichnet, daß man die Naturierung mit oxidiertem Glutathion (GSSG) und/oder GSH, Cystin und/oder Cystein, Cystamin und/oder Cysteamin durchführt.

5. Verfahren nach einem der vorhergehenden Ansprüche, dadurch gekennzeichnet, daß man ohne vorhergehende Derivatisierung die Naturierung mit Cystamin, GSSG oder Cystin bzw. den Redoxsystemen Cystamin/Cysteamin, GSH/GSSG oder Cystin/Cystein durchführt.

6. Verfahren nach einem der vorhergehenden Ansprüche, dadurch gekennzeichnet, daß man die Naturierung in Gegenwart eines Denaturierungsmittels in einer Konzentration unter 2 mol/l durchführt.

7. Verfahren nach einem der vorhergehenden Ansprüche, dadurch gekennzeichnet, daß man die Naturierung bei 2 bis 10°C und über einen Zeitraum von 2 bis 60 Stunden durchführt.

8. Verfahren nach einem der vorhergehenden Ansprüche, dadurch gekennzeichnet, daß die Naturierung als Pulsnaturierung durchgeführt wird.

9. Nach den vorhergehenden Ansprüchen rekombinant hergestellter β-NGF, dadurch gekennzeichnet, daß er weniger als 5% β-NGF-Abbauprodukte oder durch einen Met-Rest am N-Terminus modifizierten β-NGF aufweist und vorzugsweise frei ist von anderen Säugerzellproteinen.

10. Therapeutische Zusammensetzung, enthaltend als wirksame Komponente einen nach den vorhergehenden Ansprüchen rekombinant hergestellten β-NGF in Kombination mit üblichen Träger- und Hilfsstoffen.


Sequenzprotokoll

(1) ALLGEMEINE ANGABEN:

(i) ANMELDER:
(A) NAME: BOEHRINGER MANNHEIM GMBH
(B) STRASSE: Sandhofer Str. 116
(C) ORT: Mannheim
(E) LAND: Germany
(F) POSTLEITZAHL: D-68305
(G) TELEFON: 08856/60-3446
(H) TELEFAX: 08856/60-3451

(ii) BEZEICHNUNG DER ERFINDUNG: Verfahren zur gentechnologischen
Herstellung von biologisch aktivem beta-NGF

(iii) ANZAHL DER SEQUENZEN: 9

(iv) COMPUTER-LESBARE FASSUNG:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30B (EPA)

(2) ANGABEN ZU SEQ ID NO: 1:

(i) SEQUENZKENNZEICHEN:
(A) LÄNGE: 129 Aminosäuren
(B) ART: Aminosäure
(C) STRANGFORM: Einzelstrang
(D) TOPOLOGIE: linear

(ii) ART DES MOLEKÜLS: Protein

(xi) SEQUENZBESCHREIBUNG: SEQ ID NO: 1:

Met Ser Tyr Gln Arg Thr His Arg Ser Lys Arg Ser Ser Ser His Pro
1               5                   10                  15

Ile Phe His Arg Gly Glu Phe Ser Val Cys Asp Ser Val Ser Val Trp
            20                  25                  30

Val Gly Asp Lys Thr Thr Ala Thr Asp Ile Lys Gly Lys Glu Val Met
        35                  40                  45

Val Leu Gly Glu Val Asn Ile Asn Asn Ser Val Phe Lys Gln Tyr Phe
    50                  55                  60

Phe Glu Thr Lys Cys Arg Asp Pro Asn Pro Val Asp Ser Gly Cys Arg
65                  70                  75                  80

Gly Ile Asp Ser Lys His Trp Asn Ser Tyr Cys Thr Thr Thr His Thr
                85                  90                  95

Phe Val Lys Ala Leu Thr Met Asp Gly Lys Gln Ala Ala Trp Arg Phe
            100                 105                 110

Ile Arg Ile Asp Thr Ala Cys Val Cys Val Leu Ser Arg Lys Ala Val
        115                 120                 125

Arg

(2) ANGABEN ZU SEQ ID NO: 2:

(i) SEQUENZKENNZEICHEN:
(A) LÄNGE: 7 Aminosäuren
(B) ART: Aminosäure
(C) STRANGFORM: Einzelstrang
(D) TOPOLOGIE: linear

(ii) ART DES MOLEKÜLS: Peptid

(xi) SEQUENZBESCHREIBUNG: SEQ ID NO: 2:

Arg Thr His Arg Ser Lys Arg
1               5

(2) ANGABEN ZU SEQ ID NO: 3:

(i) SEQUENZKENNZEICHEN:
(A) LÄNGE: 3 Aminosäuren
(B) ART: Aminosäure
(C) STRANGFORM: Einzelstrang
(D) TOPOLOGIE: linear

(ii) ART DES MOLEKÜLS: Peptid

(xi) SEQUENZBESCHREIBUNG: SEQ ID NO: 3:

Ser Tyr Gln
1

(2) ANGABEN ZU SEQ ID NO: 4:

(i) SEQUENZKENNZEICHEN:
(A) LÄNGE: 5 Aminosäuren
(B) ART: Aminosäure
(C) STRANGFORM: Einzelstrang
(D) TOPOLOGIE: linear

(ii) ART DES MOLEKÜLS: Peptid

(xi) SEQUENZBESCHREIBUNG: SEQ ID NO: 4:

Ser Tyr Gln Val Ile
1               5

(2) ANGABEN ZU SEQ ID NO: 5:

(i) SEQUENZKENNZEICHEN:
(A) LÄNGE: 40 Basenpaare
(B) ART: Nucleotid
(C) STRANGFORM: Einzelstrang
(D) TOPOLOGIE: linear

(ii) ART DES MOLEKÜLS: other nucleic acid
(A) BESCHREIBUNG: /desc = "Primer"

(xi) SEQUENZBESCHREIBUNG: SEQ ID NO: 5:

AATTCTTATG TCTTACCAAA GAACTCACCG TAGCAAGCGC 40

(2) ANGABEN ZU SEQ ID NO: 6:

(i) SEQUENZKENNZEICHEN:
(A) LÄNGE: 40 Basenpaare
(B) ART: Nucleotid
(C) STRANGFORM: Einzelstrang
(D) TOPOLOGIE: linear

(ii) ART DES MOLEKÜLS: other nucleic acid
(A) BESCHREIBUNG: /desc = "Primer"

(xi) SEQUENZBESCHREIBUNG: SEQ ID NO: 6:

ATGAGCGCTT GCTACGGTGA GTTCTTTGGT AAGACATAAG 40

(2) ANGABEN ZU SEQ ID NO: 7:

(i) SEQUENZKENNZEICHEN:
(A) LÄNGE: 8 Aminosäuren
(B) ART: Aminosäure
(C) STRANGFORM: Einzelstrang
(D) TOPOLOGIE: linear

(ii) ART DES MOLEKÜLS: Peptid

(xi) SEQUENZBESCHREIBUNG: SEQ ID NO: 7:

Ser Tyr Gln Arg Thr His Arg Ser
1               5

(2) ANGABEN ZU SEQ ID NO: 8:

(i) SEQUENZKENNZEICHEN:
(A) LÄNGE: 8 Aminosäuren
(B) ART: Aminosäure
(C) STRANGFORM: Einzelstrang
(D) TOPOLOGIE: linear

(ii) ART DES MOLEKÜLS: Peptid

(xi) SEQUENZBESCHREIBUNG: SEQ ID NO: 8:

Gly Gln Phe Ser Val Cys Asp Ser
1               5

(2) ANGABEN ZU SEQ ID NO: 9:

(i) SEQUENZKENNZEICHEN:
(A) LÄNGE: 8 Aminosäuren
(B) ART: Aminosäure
(C) STRANGFORM: Einzelstrang
(D) TOPOLOGIE: linear

(ii) ART DES MOLEKÜLS: Peptid

(xi) SEQUENZBESCHREIBUNG: SEQ ID NO: 9:

Ser Ser Ser His Pro Ile Phe His
1               5